# EUROPEAN PATENT APPLICATION

(11) **EP 2 792 683 A1**
(43) Date of publication of application: **22.10.2014**
(21) Application number: 13382141.3
(22) Date of filing: 17.04.2013
(51) Int. Cl.: C07K 5/02, A61K 38/06, C07K 5/097, C07K 11/02, A61P 31/10

(54) **Compounds potentiating the activity of antifungal drugs**

(71) Applicant: Fundacion MEDINA. Centro de Excelencia en Investigacion de Medicamentos Innovadores en Andalucia, 18016 Armilla - Granada (ES)
(72) Inventor: Ortiz López, Francisco Javier, 18007 Granada (ES); Tormo Beltrán, José Rubén, 28002 Madrid (ES); Reyes Benítez, José Fernando, 18150 Gójar - Granada (ES); Monteiro De Aguiar, Maria Cândida, 18007 Granada (ES); Vicente Pérez, Maria Francisca, 28020 Madrid (ES); Bills, Gerald Fremont, Pearland, TX 77584 (US); González Menéndez, Victor Manuel, 18007 Granada (ES)
(74) Representative: ABG Patentes, S.L.

(57) **Abstract**

Compounds of general formula (I) 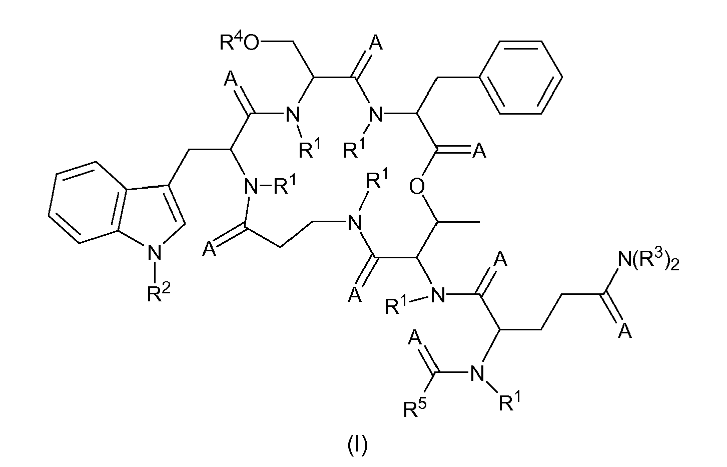 wherein R¹, R², R³, R⁴, R⁵ and A take various meanings, pharmaceutical compositions containing them and their use in medicine, particularly as agents able to potentiate the effect of antifungal drugs.

## Description

### FIELD OF THE INVENTION

The present invention relates to new compounds, pharmaceutical compositions containing them and their use in medicine, particularly as agents able to potentiate the effect of antifungal drugs.

### BACKGROUND OF THE INVENTION

Certain types of fungi (such as *Candida* spp.) are normally present on body surfaces or in the intestine. Although normally harmless, these fungi sometimes cause localized infections of the skin and nails, vagina, mouth, or sinuses. Fungi seldom cause serious harm, except in people who have a weakened immune system or who have foreign material, including medical devices (such as an intravenous catheter or an artificial joint or heart valve), in their body.

Several drugs effective against fungal infections are available such as echinocandins (caspofungin, micafungin, anidulafungin), azoles (itraconazol, voriconazol, fluconazole), or amphotericin B, but the structure and chemical makeup of fungi make them difficult to kill. Antifungal drugs may be applied directly to a fungal infection of the skin or other surface, such as the vagina or inside of the mouth. Antifungal drugs may also be taken by mouth or injected when needed to treat more serious infections. For serious infections, several months of treatment are often needed.

Synergistic effect among known antifungals and natural products is disclosed in the state of the art such as punicalagin with fluconazole **(Endo et *al.,* 2010)** and natural phenolics with amphotericin B, fluconazole and itraconazole **(Faria et *al.,* 2011).** It has also been demonstrated that the combination of echinocandins with a range of structurally diverse antimicrobial peptides results in potent synergistic killing of *Candida* spp. *in vitro* **(Harris and Coote., 2010).** As well, there are some reported cases of synergism between known antifungals such as amphotericin B with caspofungin **(Arikan et *al.,* 2002)** and triazole drugs with caspofungin **(Kiraz et *al.,* 2010).**

In certain cases fungal infections or diseases can be severe and problematic, such as opportunistic and bloodstream infections (e.g. invasive aspergillosis, candidiasis, cryptococcosis, fusariosis, trichosporonosis among other invasive fungal infections). Therefore, there is a need to obtain an antifungal therapy active and safe to be administered to patients suffering, or likely to suffer, from a fungal disease. The problem to be solved by the present invention is to provide compounds that are useful in the therapy of fungal diseases.

### BRIEF DESCRIPTION OF THE INVENTION

The present invention solves the aforementioned need by the provision of new compounds able to potentiate the effect of antifungal drugs.

In one aspect, the present invention is directed to compounds of general formula (I) or pharmaceutically acceptable salts, isomers, prodrugs or solvates thereof 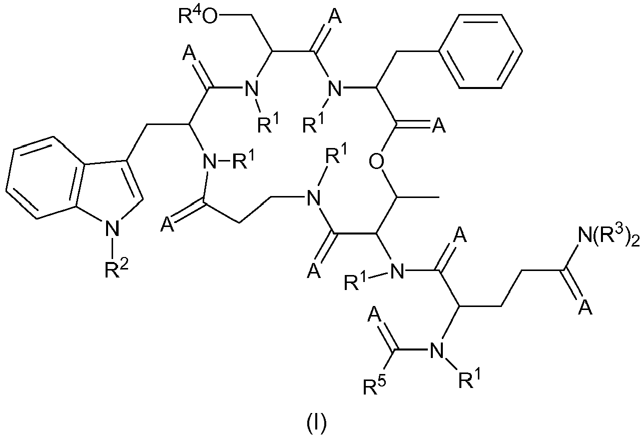 wherein
each R¹, R² and R³ are independently selected from hydrogen and an amino protecting group;
R⁴ is selected from hydrogen and a hydroxyl protecting group;
R⁵ is selected from hydrogen, ORₐ, OCORₐ, NRₐR_{b}, substituted or unsubstituted C₁-C₁₈ alkyl, substituted or unsubstituted C₂-C₁₈ alkenyl, substituted or unsubstituted C₂-C₁₈ alkynyl, and substituted or unsubstituted aryl;
each A is independently selected from oxygen and sulphur; and
each Rₐ and R_{b} is independently selected from hydrogen, substituted or unsubstituted C₁-C₁₂ alkyl, substituted or unsubstituted C₂-C₁₂ alkenyl, substituted or unsubstituted C₂-C₁₂ alkynyl, and substituted or unsubstituted aryl.

Another aspect of this invention refers to a medicament or pharmaceutical composition comprising at least one compound of formula (I) as defined above, or a pharmaceutically acceptable salt, isomer, prodrug or solvate thereof and a pharmaceutically acceptable excipient.

Another aspect of this invention refers to a compound of formula (I) as defined above, or a pharmaceutically acceptable salt, isomer, prodrug or solvate thereof for use in medicine, particularly for the treatment and/or prophylaxis of a fungal disease.

Another aspect of this invention refers to the use of a compound of formula (I) as defined above, or a pharmaceutically acceptable salt, isomer, prodrug or solvate thereof in the manufacture of a medicament for the treatment and/or prophylaxis of a fungal disease.

Another aspect of the present invention refers to a method for the treatment and/or prophylaxis of a fungal disease, the method comprising administering to the subject in need of such a treatment or prophylaxis an effective amount of a compound of formula (I) as defined above, or a pharmaceutically acceptable salt, isomer, prodrug or solvate thereof.

The present invention establishes that the lipopeptide compounds of general formula (I) or pharmaceutically acceptable salts, isomers, prodrugs or solvates thereof potentiate the activity of antifungal drugs. Therefore, the compound of formula (I) and said antifungal drug can be successfully used in combination therapy for the treatment of a fungal disease. Thus, this invention is directed to pharmaceutical compositions, kits, methods for the treatment of fungal diseases using these combination therapies and uses of both drugs in the treatment of fungal diseases and in the manufacture of medicaments for combination therapies.

The present invention also relates to the obtention of compounds of formula (I) from microorganisms capable of producing them, specifically from a fungus identified as *Colispora cavincola,* and more specifically from the strains of C. *cavincola* CBS 133614 and C. *cavincola* CBS 624.95, and the formation of derivatives from the obtained compounds. In a preferred embodiment, the process for obtaining compounds of formula (I) comprises the steps of cultivating a strain of a microorganism capable of producing them such as C. *cavincola* CBS 133614 or C. *cavincola* CBS 624.95 in an aqueous nutrient medium with assimilable carbon and nitrogen sources and salts, under controlled submerged aerobic conditions, and then recovering and purifying the compounds according to the invention from the cultured broth.

Another aspect of the invention is the strain of *Colispora cavincola* with accession number CBS 133614.

These aspects and preferred embodiments thereof are additionally also defined hereinafter in the detailed description, as well as in the claims.

### BRIEF DESCRIPTION OF THE FIGURES

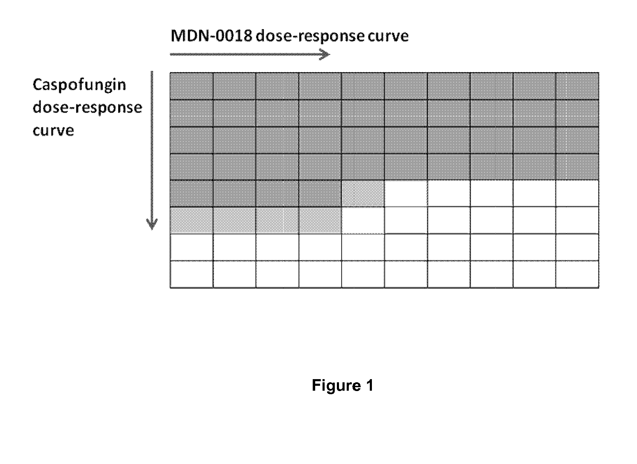

**Figure 1****.** Checkerboard assay showing the potentiation of the antifungal caspofungin by the compound MDN-0018. In this assay, dose response curves of the compound MDN-0018 (8 to 0.015 µg/mL) are performed from left to right and dose response curves of caspofungin (0.24 to 0.018 µg/mL) are performed from top to bottom. Other known antifungal compounds (echinocandins, azoles and polyenes) were tested, obtaining a similar checkboard. The result shows how much the peptide MDN-0018 potentiates the effect of the known antifungal by decreasing its minimal inhibitory concentration (MIC).

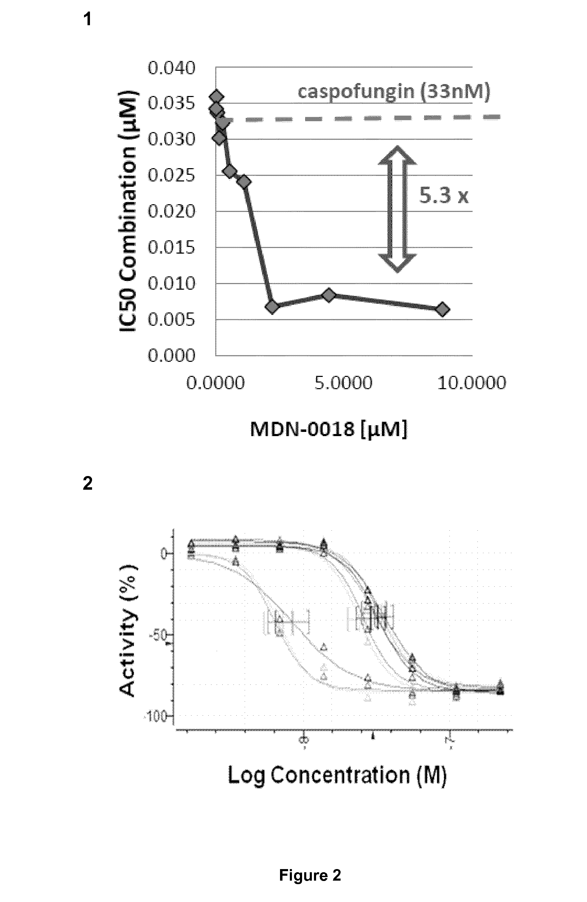

**Figure 2****.** Determination of the potentiation of caspofungin activity by the compound MDN-0018 against *A. fumigatus.* Study of the optimum concentration of the compounds MDN-0018 and caspofungin where maximum enhancement of caspofungin is reached is shown in Figure 2.1. In addition, the graphic representation of curves, in duplicate, of the caspofungin potentiated by the compound MDN-0018 is shown in Figure 2.1. Each curve represents a curve of caspofungin from 0.24 to 0.018 µg/mL (0.22 to 0.0017 µM) with set concentrations of the MDN-0018 compound from 8 to 0.015 µg/mL (8.86 to 0.017 µM).

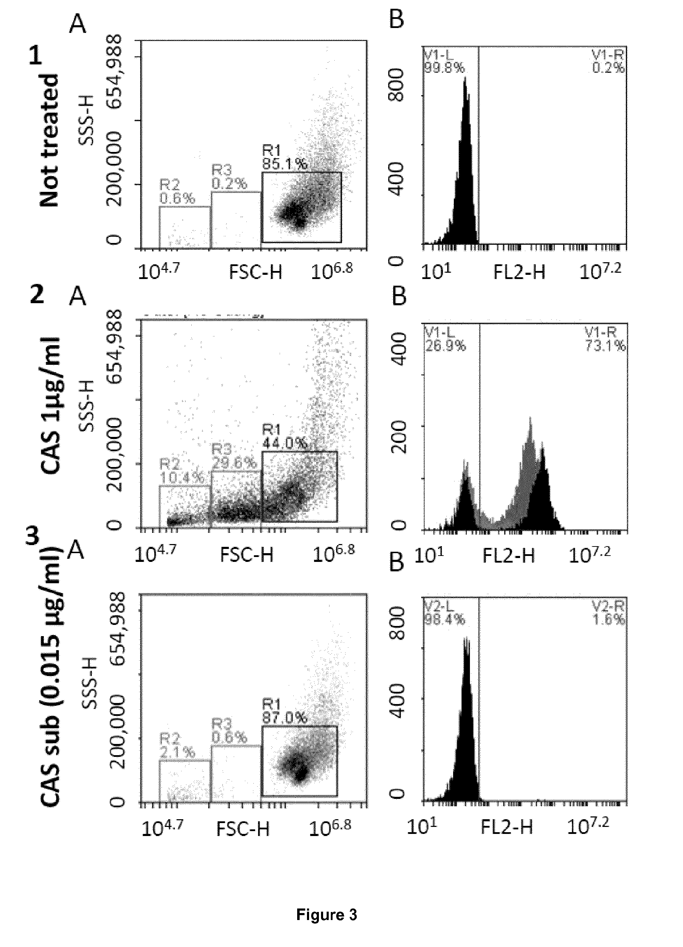

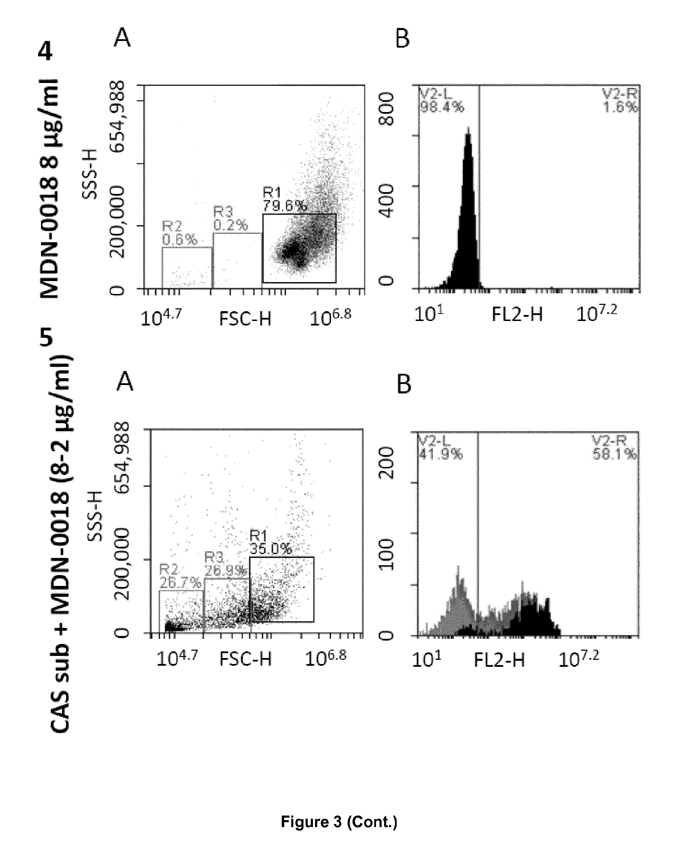

**Figure 3****.** Flow cytometry experiments to determine cellular viability by changes in membrane permeability using propidium iodide (PI) as probe. The antifungal effects of the compound were evaluated with C. *albicans* and demonstrated that the sublethal dose of caspofungin or the compound MDN-0018 used at concentrations below 8 µg/mL do not affect the cellular viability 1) Not treated cells, (1A) shows the normal population and (1B) shows the background of fluorescence emitted. 2) Cells treated with 1 µg/mL of caspofungin. (2A) shows the damaged population and (2B) confirmed that PI was able to enter into the cell. 3) The cells treated with the sublethal dose of caspofungin (0.015 µg/mL) (3A and 3B) presented the same profile as the non treated cells (healthy cells) as well as 4) (4A and 4B) the cells treated with 8 µg/mL of the MDN-0018 compound. 5) The cells treated with the sublethal dose of caspofungin plus the compound MDN-0018 at concentrations from 8 to 2 µg/mL (5A and 5B) presented the same damage profile as the cells treated with 1 µg/mL of caspofungin.

### DETAILED DESCRIPTION OF THE INVENTION

The efficacy of the active components can sometimes be improved by addition of other (active) ingredients. More rarely, the observed efficacy of the combination of ingredients can be significantly higher than what would be expected from the amounts of the individual ingredients used, thus indicating potentiation of the activity of the components of the combination.

The present inventors have surprisingly found that lipopeptide compounds of general formula (I) are able to potentiate the effect of antifungal drugs. Thus, the present invention relates to compounds of general formula (I) as defined above.

In these compounds the groups can be selected in accordance with the following guidance:

Alkyl groups may be branched or unbranched, and preferably have from 1 to about 18 carbon atoms, such as from 1 to about 12 carbon atoms. One more preferred class of alkyl groups has from 6 to about 18 carbon atoms; even more preferred are alkyl groups having 8, 9, 10, 11, 12, 13 or 14 carbon atoms. Another more preferred class of alkyl groups has from 1 to about 6 carbon atoms; even more preferred are alkyl groups having 1, 2, 3 or 4 carbon atoms. Methyl, ethyl, n-propyl, iso-propyl and butyl, including n-butyl, *tert*-butyl, *sec*-butyl and iso-butyl as well as branched or unbranched octyl, nonyl, decyl, undecyl, dodecyl, tridecyl and tetradecyl are particularly preferred alkyl groups in the compounds of the present invention. As used herein, the term alkyl, unless otherwise stated, refers to both cyclic and non-cyclic groups, although cyclic groups will comprise at least three carbon ring members.

Alkenyl and alkynyl groups in the compounds of the present invention may be branched or unbranched, have one or more unsaturated linkages and from 2 to about 18 carbon atoms, such as from 2 to about 12 carbon atoms. One more preferred class of alkenyl and alkynyl groups has from 6 to about 18 carbon atoms; even more preferred are alkenyl and alkynyl groups having 8, 9, 10, 11, 12, 13 or 14 carbon atoms. Another more preferred class of alkenyl and alkynyl groups has from 2 to about 6 carbon atoms; even more preferred are alkenyl and alkynyl groups having 2, 3 or 4 carbon atoms. The terms alkenyl and alkynyl as used herein refer to both cyclic and noncyclic groups, although cyclic groups will comprise at least three carbon ring members.

Suitable aryl groups in the compounds of the present invention include single and multiple ring compounds, including multiple ring compounds that contain separate and/or fused aryl groups. Typical aryl groups contain from 1 to 3 separated and/or fused rings and from 6 to about 18 carbon ring atoms. Preferably aryl groups contain from 6 to about 10 carbon ring atoms. Specially preferred aryl groups include substituted or unsubstituted phenyl, substituted or unsubstituted naphthyl, substituted or unsubstituted biphenyl, substituted or unsubstituted phenanthryl and substituted or unsubstituted anthryl.

The groups above mentioned may be substituted at one or more available positions by one or more suitable groups such as OR', =O, SR', SOR', SO₂R', OSO₂R', OSO₃R', NO₂, NHR', N(R')₂, =N-R', N(R')COR', N(COR')₂, N(R')SO₂R', N(R')C(=NR')N(R')R', CN, halogen, COR', COOR', OCOR', OCOOR', OCONHR', OCON(R')₂, CONHR', CON(R')₂, CON(R')OR', CON(R')SO₂R', PO(OR')₂, PO(OR')R', PO(OR')(N(R')R'), substituted or unsubstituted C₁-C₁₂ alkyl, substituted or unsubstituted C₂-C₁₂ alkenyl, substituted or unsubstituted C₂-C₁₂ alkynyl, and substituted or unsubstituted aryl, wherein each of the R' groups is independently selected from the group consisting of hydrogen, OH, NO₂, NH₂, SH, CN, halogen, COH, COalkyl, COOH, substituted or unsubstituted C₁-C₁₂ alkyl, substituted or unsubstituted C₂-C₁₂ alkenyl, substituted or unsubstituted C₂-C₁₂ alkynyl, and substituted or unsubstituted aryl. Where such groups are themselves substituted, the substituents may be chosen from the foregoing list.

Suitable halogen groups or substituents in the compounds of the present invention include F, Cl, Br, and I.

Suitable protecting groups are well known for the skilled person in the art. A general review of protecting groups in organic chemistry is provided by Wuts, PGM and Greene TW in Protecting Groups in Organic Synthesis, 4th Ed. Wiley-Interscience, and by Kocienski PJ in Protecting Groups, 3rd Ed. Georg Thieme Verlag. These references provide sections on protecting groups for hydroxy and amino groups. All these references are incorporated by reference in their entirety.

Examples of such protected OH include ethers, silyl ethers, esters, sulfonates, sulfenates and sulfinates, carbonates and carbamates. In the case of ethers the protecting group for the OH can be selected from methyl, methoxymethyl, methylthiomethyl, (phenyldimethylsilyl)methoxymethyl, benzyloxymethyl, *p-*methoxybenzyloxymethyl, [(3,4-dimethoxybenzyl)oxy]methyl, *p*-nitrobenzyloxymethyl, *o-*nitrobenzyloxymethyl, [(*R*)-1-(2-nitrophenyl)ethoxy]methyl, (4-methoxyphenoxy)methyl, guaiacolmethyl, [(*p*-phenylphenyl)oxy]methyl, *t*-butoxymethyl, 4-pentenyloxymethyl, siloxymethyl, 2-methoxyethoxymethyl, 2-cyanoethoxymethyl, bis(2-chloroethoxy)methyl, 2,2,2-trichloroethoxymethyl, 2-(trimethylsilyl)ethoxymethyl, menthoxymethyl, *o*-bis(2-acetoxyethoxy)methyl, tetrahydropyranyl, fluorous tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl *S,S*-dioxide, 1-[(2-chloro-4-methyl)phenyl]-4-methoxypiperidin-4-yl, 1-(2fluorophenyl)-4-methoxypiperidin-4-yl, 1-(4-chlorophenyl)-4-methoxypiperidin-4-yl, 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 2-hydroxyethyl, 2-bromoethyl, 1-[2-(trimethylsilyl)ethoxy]ethyl, 1-methyl-1-methoxyethyl, 1-methyl-1-benzyloxyethyl, 1-methyl-1-benzyloxy-2-fluoroethyl, 1-methyl-1-phenoxyethyl, 2,2,2-trichloroethyl, 1,1-dianisyl-2,2,2-trichloroethyl, 1,1,1,3,3,3-hexafluoro-2-phenylisopropyl, 1-(2-cyanoethoxy)ethyl, 2-trimethylsilylethyl, 2-(benzylthio)ethyl, 2-phenylselenyl)ethyl, *t*-butyl, cyclohexyl, 1-methyl-1'-cyclopropylmethyl, allyl, prenyl, cinnamyl, 2-phenallyl, propargyl, *p*-chlorophenyl, *p*-methoxyphenyl, *p*-nitrophenyl, 2,4-dinitrophenyl, 2,3,5,6-tetrafluoro-4-(trifluoromethyl)phenyl, benzyl, p-methoxybenzyl, 3,4-dimethoxybenzyl, 2,6-dimethoxybenzyl, *o*-nitrobenzyl, *p*-nitrobenzyl, pentadienylnitrobenzyl, pentadienylnitropiperonyl, halobenzyl, 2,6-dichlorobenzyl, 2,4-dichlorobenzyl, 2,6-difluorobenzyl, *p*-cyanobenzyl, fluorous benzyl, 4-fluorousalkoxybenzyl, trimethylsilylxylyl, *p*-phenylbenzyl, 2-phenyl-2-propyl, *p-*acylaminobenzyl, *p*-azidobenzyl, 4-azido-3-chlorobenzyl, 2-trifluoromethylbenzyl, 4-trifluoromethylbenzyl, *p*-(methylsulfinyl)benzyl, *p*-siletanylbenzyl, 4-acetoxybenzyl, 4-(2-trimethylsilyl)ethoxymethoxybenzyl, 2-naphthylmethyl, 2-picolyl, 4-picolyl, 3-methyl-2-picolyl *N-*oxido, 2-quinolinylmethyl, 6-methoxy-2-(4-methylphenyl-4-quinolinemethyl, 1-pyrenylmethyl, diphenylmethyl, 4-methoxydiphenylmethyl, 4-phenyldiphenylmethyl, *p,p*'-dinitrobenzhydryl, 5-dibenzosuberyl, triphenylmethyl, tris(4-*t*-butylphenyl)methyl, α-naphthyldiphenylmethyl, *p-*methoxyphenyldiphenylmethyl, di(*p*-methoxyphenyl)phenylmethyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 4,4'-dimethoxy-3"-[*N*-(imidazolylmethyl)]trityl, 4,4'-dimethoxy-3"-[*N*-(imidazolylethyl)carbamoyl]trityl, bis(4-methoxyphenyl)-1'-pyrenylmethyl, 4-(17-tetrabenzo[*a,c,g*,i]fluorenylmethyl)4,4"-dimethoxytrityl, 9-anthryl, 9-(9-phenyl)xanthenyl, 9-phenylthioxanthyl, 9-(9-phenyl-10-oxo)anthryl, 1,3-benzodithiolan-2-yl, and 4,5-bis(ethoxycarbonyly[1,3]-dioxolan-2-yl, benzisothiazolyl *S,S*-dioxido. In the case of silyl ethers the protecting group for the OH can be selected from trimethylsilyl, triethylsilyl, triisopropylsilyl, dimethylisopropylsilyl, diethylisopropylsilyl, dimethylhexylsylil, 2-norbomyldimethylsilyl, *t*-butyldimethylsilyl, *t*-butyldiphenylsilyl, tribenzylsilyl, tri-*p*-xylylsilyl, triphenylsilyl, diphenylmethylsilyl, di-*t*-butylmethylsilyl, bis(*t*-butyly)-1-pyrenylmethoxysilyl, tris(trimethylsilyl)silyl, (2-hydroxystyryl)dimethylsilyl, (2-hydroxystyryl)diisopropylsilyl, *t*-butylmethoxyphenylsilyl, *t*-butoxydiphenylsilyl, 1,1,3,3-tetraisopropyl-3-[2-(triphenylmethoxy)ethoxy]disiloxane-1-yl, and fluorous silyl. In the case of esters the protecting group for the OH can be selected from formate, benzoylformate, acetate, chloroacetate, dichloroacetate, trichloroacetate, trichloroacetamidate, trifluoroacetate, methoxyacetate, triphenylmethoxyacetate, phenoxyacetate, *p*-chlorophenoxyacetate, phenylacetate, diphenylacetate, 3-phenylpropionate, bisfluorous chain type propanoyl, 4-pentenoate, 4-oxopentanoate, 4,4-(ethylenedithio)pentanoate, 5[3-bis(4-methoxyphenyl)hydroxymethylphenoxy]levulinate, pivaloate, 1-adamantoate, crotonate, 4-methoxycrotonate, benzoate, *p*-phenylbenzoate, 2,4,6-trimethylbenzoate, 4-bromobenzoate, 2,5-difluorobenzoate, p-nitrobenzoate, picolinate, nicotinate, 2-(azidomethyl)benzoate, 4-azidobutyrate, (2-azidomethyl)phenylacetate, 2-{[(tritylthio)oxy]methyl}benzoate, 2-{[(4-methoxytritylthio)oxy]methyl}benzoate, 2-{[methyl(tritylthio)amino]methyl}benzoate, 2-{{[(4-methoxytrityl)thio]methylamino}-methyl}benzoate, 2-(allyloxy)phenylacetate, 2-(prenyloxymethyl)benzoate, 6-(levulinyloxymethyl)-3-methoxy-2-nitrobenzoate, 6-(levulinyloxymethyl)-3-methoxy-4-nitrobenzoate, 4-benzyloxybutyrate, 4-trialkylsilyloxybutyrate, 4-acetoxy-2,2-dimethylbutyrate, 2,2-dimethyl-4-pentenoate, 2-iodobenzoate, 4-nitro-4-methylpentanoate, *o*-(dibromomethyl)benzoate, 2-formylbenzenesulfonate, 4-(methylthiomethoxy)butyrate, 2-(methylthiomethoxymethyl)benzoate, 2-(chloroacetoxymethyl)benzoate, 2-[(2-chloroacetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, *o*-(methoxycarbonyl)benzoate, α-naphthoate, nitrate, alkyl *N,N,N,N-*tetramethylphosphorodiamidate, and 2-chlorobenzoate. In the case of sulfonates, sulfenates and sulfinates the protecting group for the OH can be selected from sulfate, allylsulfonate, methanesulfonate, benzylsulfonate, tosylate, 2-[(4-nitrophenyl)ethyl]sulfonate, 2-trifluoromethylbenzenesulfonate, 4-monomethoxytritylsulfenate, alkyl 2,4-dinitrophenylsulfenate, 2,2,5,5-tetramethylpyrrolidin-3-one-1-sulfinate, borate, and dimethylphosphinothiolyl. In the case of carbonates the protecting group for the OH can be selected from methyl carbonate, methoxymethyl carbonate, 9-fluorenylmethyl carbonate, ethyl carbonate, bromoethyl carbonate, 2-(methylthiomethoxy)ethyl carbonate, 2,2,2-trichloroethyl carbonate, 1,1-dimethyl-2,2,2-trichloroethyl carbonate, 2-(trimethylsilyl)ethyl carbonate, 2-[dimethyl(2-naphthylmethyl)silyl]ethyl carbonate, 2-(phenylsulfonyl) ethyl carbonate, 2-(triphenylphosphonio)ethyl carbonate, cis-[4-[[(methoxytrityl)sulfenyl]oxy]tetrahydrofuran-3-yl]oxy carbonate, isobutyl carbonate, *t*-butyl carbonate, vinyl carbonate, allyl carbonate, cinnamyl carbonate, propargyl carbonate, p-chlorophenyl carbonate, *p*-nitrophenyl carbonate, 4-ethoxy-1-naphthyl carbonate, 6-bromo-7-hydroxycoumarin-4-yimethyl carbonate, benzyl carbonate, *o*-nitrobenzyl carbonate, *p*-nitrobenzyl carbonate, *p*-methoxybenzyl carbonate, 3,4-dimethoxybenzyl carbonate, anthraquinon-2-ylmethyl carbonate, 2-dansylethyl carbonate, 2-(4-nitrophenyl)ethyl carbonate, 2-(2,4-dinitrophenyl)ethyl carbonate, 2-(2-nitrophenyl)propyl carbonate, alkyl 2-(3,4-methylenedioxy-6-nitrophenyl)propyl carbonate, 2-cyano-1-phenylethyl carbonate, 2-(2-pyridyl)amino-1-phenylethyl carbonate, 2-[*N*-methyl-*N*-(2-pyridyl)]amino-1-phenylethyl carbonate, phenacyl carbonate, 3',5'-dimethoxybenzoin carbonate, methyl dithiocarbonate, and S-benzyl thiocarbonate. And in the case of carbamates the protecting group for the OH can be selected from dimethylthiocarbamate, *N*-phenylcarbamate, *N*-methyl-*N*-(*o*-nitrophenyl)carbamate. The mention of these groups should be not interpreted as a limitation of the scope of the invention, since they have been mentioned as a mere illustration of protecting groups for OH, but further groups having said function may be known by the skilled person in the art, and they are to be understood to be also encompassed by the present invention.

Examples of protected amino groups include carbamates, ureas, amides, heterocyclic systems, *N*-alkyl amines, *N*-alkenyl amines, *N*-alkynyl amines, *N*-aryl amines, imines, enamines, N-metal derivatives, N-N derivatives, N-P derivatives, N-Si derivatives, and N-S derivatives. In the case of carbamates the protecting group for the amino group can be selected from methylcarbamate, ethylcarbamate, 9-fluorenylmethyl-carbamate, 2,6-di-*t*-butyl-9-fluorenylmethylcarbamate, 2,7-bis(trimethylsilyl)fluorenylmethylcarbamate, 9-(2-sulfo)fluorenylmethyl carbamate, 9-(2,7-dibromo)fluorenylmethylcarbamate, 17-tetrabenzo[*a,c,g,i*]fluorenylmethylcarbamate, 2-chloro-3-indenylmethyl-carbamate, benz[*f*]inden-3-ylmethylcarbamate, 1,1-dioxobenzo[*b*]thiophene-2-ylmethylcarbamate, 2-methylsulfonyl-3-phenyl-1-prop-2-enyloxycarbamate, 2,7-di-*t*-butyl-[9,(10,10-dioxo-10,10,10,10-tetrahydrothioxanthyl)]methylcarbamate, 2,2,2-trichloroethylcarbamate, 2-trimethylsilylethylcarbamate, (2-phenyl-2-trimethylsilyl)ethylcarbamate, 2-phenylethylcarbamate, 2-chloroethylcarbamate, 1,1-dimethyl-2-haloethylcarbamate, 1,1-dimethyl-2,2-dibromoethylcarbamate, 1,1-dimethyl-2,2,2-trichloroethylcarbamate, 2-(2'-pyridyl)ethylcarbamate, 2-(4'-pyridyl)ethylcarbamate, 2,2-bis(4'-nitrophenyl)ethylcarbamate, 2-[(2-nitrophenyl)dithio]-1-phenylethylcarbamate, *2-(N,N-*dicyclohexyl-carboxamido)ethylcarbamate, *t*-butylcarbamate, C₈F₁₉CH₂CH₂C(CH₃)₂-carbamate, 1-adamantylcarbamate, 2-adamantyl carbamate, 1-(1-adamantyly)-methylethylcarbamate, 1-methyl-1-(4-byphenylyl)ethylcarbamate, 1-(3,5-di-*t-*butylphenyl)-1-methyl-ethylcarbamate, triisopropylsiloxylcarbamate, vinylcarbamate, allylcarbamate, prenylcarbamate, 1-isopropylallylcarbamate, cinnamylcarbamate, 4-nitrocinnamylcarbamate, 3-(3'-pyridyl)prop-2-enylcarbamate, hexadienyloxycarbamate, propargyloxycarbamate, but-2-ynylbisoxycarbamate, 8-quinolylcarbamate, *N*-hydroxypiperidinyl-carbamate, alkyldithiocarbamate, benzylcarbamate, 3,5-di-*t*-butylbenzylcarbamate, p-methoxybenzylcarbamate, *p*-nitrobenzylcarbamate, *p*-bromobenzylcarbamate, *p*-chlorobenzyl-carbamate, 2,4-dichlorobenzylcarbamate, 4-methylsulfinylbenzyl-carbamate, 4-trifluoromethylbenzylcarbamate, C₈F₁₇CH₂CH₂-carbamate, (C₈F₁₇CH₂CH₂)₃Si-carbamate, 2-naphthylmethylcarbamate, 9-anthryl-methylcarbamate, diphenylmethylcarbamate, 4-phenylacetoxybenzyl-carbamate, 4-azidobenzylcarbamate, 4-azidomethoxybenzylcarbamate, *m*-chloro-*p*-acyloxybenzylcarbamate, *p*-(dihydroxyboryl)benzylcarbamate, 5-benzisoxazolylmethylcarbamate, 2-(trif)uoromethyl)-6-chromonylmethyl-carbamate, 2-methylthioethylcarbamate, 2-methylsulfonylethylcarbamate, 2-(*p*-toluenesulfonyl)ethylcarbamate, 2-(4-nitrophenylsulfonyl)ethoxy-carbamate, 2-(2,4-dinitrophenylsulfonyl)ethoxycarbamate, 2-(4-trifluoromethylphenylsulfonyl)ethoxycarbamate, [2-(1,3-dithianyl)]methyl-carbamate, 2-phosphonioethylcarbamate, 2-[phenyl(methyl)sulfonio]ethyl-carbamate, 1-methyl-1-(triphenylphosphonio)ethylcarbamate, 1,1-dimethyl-2-cyanoethylcarbamate, 2-dansylethylcarbamate, 2-(4-nitrophenyl)ethylcarbamate, 4-methylthiophenylcarbamate, 2,4-dimethylthiophenylcarbamate, m-nitrophenylcarbamate, 3,5-dimethoxy-benzylcarbamate, 1-methyl-1-(3,5-dimethoxyphenyl)ethylcarbamate, α-methylnitropiperonylcarbamate, *o-*nitrobenzylcarbamate, 3,4-dimethoxy-6-nitrobenzylcarbamate, phenyl(*o-*nitrophenyl)methylcarbamate, 2-nitrophenylethylcarbamate, 6-nitroveratrylcarbamate, 4-methoxyphenacyl-carbamate, 3',5'-dimethoxybenzoincarbamate, 9-xanthenylmethyl-carbamate, *N*-methyl-*N*-(*o*-nitrophenyl)carbamate, *N*-(2-acetoxyethyl)-aminecarbamate, *t*-amylcarbamate, 1-methylcyclobutylcarbamate, 1-methylcyclohexylcarbamate, 1-methyl-1-cyclopropylmethylcarbamate, cyclobutylcarbamate, cyclopentylcarbamate, cyclohexylcarbamate, isobutylcarbamate, isobomylcarbamate, cyclopropylmethylcarbamate, p-decyloxybenzylcarbamate, diisopropylmethylcarbamate, 2,2-dimethoxy-carbonylvinylcarbamate, *o*-(*N,N*-dimethylcarboxamido)benzylcarbamate, 1,1-dimethyl-3-(*N,N-*dimethylcarboxamido)propylcarbamate, butynyl-carbamate, 1,1-dimethylpropynylcarbamate, 2-iodoethylcarbamate, 1-methyl-1-(4'-pyridyl)ethylcarbamate, 1-methyl-1-(*p-*phenylazophenyl)ethylcarbamate, *p*-(*p*'-methoxyphenylazo)benzylcarbamate, *p*-(phenylazo)benzyl-carbamate, 2,4,6-trimethylbenzylcarbamate, isonicotinylcarbamate, 4-(trimethyl-ammonium)benzylcarbamate, *p-*cyanobenzylcarbamate, di(2-pyridyl)methylcarbamate, 2-furanylmethylcarbamate, phenylcarbamate, 2,4,6-tri-*t*-butylphenylcarbamate, 1-methyl-1-phenylethylcarbamate, and S-benzyl thiocarbamate. In the case of ureas the protecting groups for the amino group can be selected from phenothiazinyl-(10)-carbonyl, *N*'-*p*-toluenesulfonylaminocarbonyl, *N*'-phenylaminothio-carbonyl, 4-hydroxyphenylaminocarbonyl, 3-hydroxytryptaminocarbonyl, and *N*'-phenyl-aminothiocarbonyl. In the case of amides the protecting group for the amino group can be selected from formamide, acetamide, chloroacetamide, trichloroacetamide, trifluoroacetamide, phenylacetamide, 3-phenylpropanamide, pent-4-enamide, picolinamide, 3-pyridyl-carboxamide, *N*-benzoylphenylalanyl, benzamide, *p*-phenylbenzamide, *o*-nitrophenylacetamide, 2,2-dimethyl-2-(o-nitrophenyl)acetamide, *o*-nitrophenoxyacetamide, 3-(o-nitrophenyl)propanamide, 2-methyl-2-(*o*-nitrophenoxy)propanamide, 3-methyl-3-nitrobutanamide, *o*-nitrocinnamide, *o-*nitrobenzamide, 3-(4-*t*-butyl-2,6-dinitrophenyl)-2,2-dimethylpropanamide, *o-*benzoyloxymethyl)benzamide, 2-(acetoxymethyl)-benzamide, 2-[(*t-*butyldiphenylsiloxy)methyl]benzamide, 3-(3',6'-dioxo-2',4',5'-trimethylcyclohexa-1',4'-diene)-3,3-dimethylpropionamide, *o*-hydroxy-*trans*-cinnamide, 2-methyl-2-(*o-*phenylazophenoxy)propanamide, 4-chlorobutanamide, acetoacetamide, 3-(p-hydroxyphenyl)propanamide, (*N*'-dithiobenzyloxycarbonylamino)acetamide, and *N-*acetylmethionine amide. In the case of heterocyclic systems the protecting group for the amino group can be selected from 4,5-diphenyl-3-oxazolin-2-one, *N*-phthalimide, *N*-dichlorophthalimide, *N*-tetrachlorophthalimide, *N*-4-nitrophthalimide, *N-*thiodiglycoloyl, *N*-dithiasuccinimide, *N*-2,3-diphenylmaleimide, *N*-2,3-dimethylmaleimide, *N*-2,5-dimethylpyrrole, *N*-2,5-bis(triisopropylsiloxy)pyrrole, *N*-1,1,4,4-tetramethyldisilylazacyclo-pentane adduct, *N-*1,1,3,3-tetramethyl-1,3-disilaisoindoline, *N-*diphenylsilyldiethylene, *N*-5-substituted-1,3-dimethyl-1,3,5-triazacyclohexan-2-one, *N*-5-substituted -1,3-benzyl-1,3,5-triazacyclohexan-2-one, 1-substituted 3,5-dinitro-4-pyridone, and 1,3,5-dioxazine. In the case of *N*-alkyl, *N*-alkenyl, *N*-alkynyl or *N*-aryl amines the protecting group for the amino group can be selected from *N*-methyl, *N-t*-butyl, *N-*allyl, *N*-prenyl, *N*-cinnamyl, *N*-phenylallyl, *N*-propargyl, *N*-methoxymethyl, *N-[2-*(trimethylsilyl)ethoxy]methyl, *N*-3-acetoxypropyl, *N*-cyanomethyl, *N*-2-azanorbomenes, *N*-benzyl, *N*-4-methoxybenzyl, *N*-2,4-dimethoxybenzyl, *N*-2-hydroxybenzyl, *N*-ferrocenylmethyl, *N-*2,4-dinitrophenyl, *o*-methoxyphenyl, *p*-methoxyphenyl, *N*-9-phenylfluorenyl, *N*-fluorenyl, *N*-2-picolylamine N'-Oxide, *N*-7-methoxycoumar-4-ylmethyl, *N*-diphenylmethyl, *N*-bis(4-methoxyphenyl)methyl, *N*-5-dibenzosuberyl, *N*-triphenylmethyl, *N-(*4-methylphenyl)diphenylmethyl, and *N*-(4-methoxyphenyl)diphenylmethyl. In the case of imines the protecting group for the amino group can be selected from *N*-1,1-dimethylthiomethylene, *N-*benzylidene, *N*-*p*-methoxybenzylidene, *N*-diphenylmethylene, *N*-[2-pyridyl)mesityl]methylene, *N-*(*N',N'*-dimethylaminomethylene), *N-(N',N'*-dibenzylaminomethylene), *N-(N'-t-*butylaminomethylene), *N,N'*-isopropylidene, *N-p*-nitrobenzylidene, *N*-salicylidene, *N-5-*chlorosalicylidene, *N*-(5-chloro-2-hydroxyphenyl)phenylmethylene, *N-*cyclohexylidene, and *N-t-*butylidene. In the case of enamines the protecting group for the amino group can be selected from *N*-(5,5-dimethyl-3-oxo-1-cyclohexenyl), *N*-2,7-dichloro-9-fluorenylmethylene, *N-*1*-(4,4-*dimethyl-2,6-dioxocyclohexylidene)ethyl, *N*-(1,3-dimethyl-2,4,6-(1*H*,3*H*,5*H*)-trioxopyrimidine-5-ylidene)methyl, *N*-4,4,4-trifluoro-3-oxo-1-butenyl, and *N*-(1-isopropyl-4-nitro-2-oxo-3-pyrrolin-3-yl). In the case of N-metal derivatives the protecting group for the amino group can be selected from N-borane, *N*-diphenyiborinic acid, *N*-diethylborinic acid, *N*-9-borabicyclononane, *N-*difluoroborinic acid, and 3,5-bis(trifluoromethyl)phenylboronic acid; and also including *N-*[phenyl(pentacarbonylchromium)]carbenyl, *N*-[phenyl(pentacarbonyl-tungsten)]carbenyl, *N-*[methyl(pentacarbonylchromium)]carbenyl, *N-*[methyl(pentacarbonyltungsten)]-carbenyl, *N-*copper chelate, *N*-zinc chelate, and a 18-crown-6-derivative. In the case of N-N derivatives the protecting group for the amino group can be selected from *N*-nitro, *N*-nitroso, *N-*oxide, azide, triazene, and *N*-trimethylsilylmethyl-*N*-benzylhydrazine. In the case of N-P derivatives the protecting group for the amino group can be selected from diphenylphosphinamide, dimethylthiophosphinamide, diphenylthiophosphinamide, dialkyl phosphoramidate, dibenzyl phosphoramidate, diphenyl phosphoramidate, and iminotriphenylphosphorane. In the case of N-Si derivatives the protecting group for the NH₂ can be selected from t-butyldiphenylsilyl and triphenylsilyl. In the case of N-S derivatives the protecting group for the amino group can be selected from N-sulfenyl or N-sulfonyl derivatives. The N-sulfenyl derivatives can be selected from benzenesulfenamide, 2-nitrobenzenesulfenamide, 2,4-dinitrobenzenesulfenamide, pentachloro-benzenesulfenamide, 2-nitro-4-methoxybenzenesulfenamide, triphenyl-methylsulfenamide, 1-(2,2,2)-trifluoro-1,1-diphenyl)ethylsulfenamide, and *N*-3-nitro-2-pyridinesulfenamide. The N-sulfonyl derivatives can be selected from methanesulfonamide, trifluoromethanesulfonamide, *t*-butylsulfonamide, benzylsulfonamide, 2-(trimethylsilyl)ethanesulfonamide, *p*-toluenesulfonamide, benzenesulfonamide, *o-*anisylsulfonamide, 2-nitrobenzenesulfonamide, 4-nitrobenzenesulfonamide, 2,4-dinitrobenzenesulfonamide, 2-naphthalenesulfonamide, 4-(4',8'-dimethoxynaphthylmethyl)benzenesulfonamide, 2-(4-methylphenyl)-6-methoxy-4-methylsulfonamide, 9-anthracenesulfonamide, pyridine-2-sulfonamide, benzothiazole-2-sulfonamide, phenacylsulfonamide, 2,3,6-trimethyl-4-methoxybenzenesulfonamide, 2,4,6-trimethoxybenzene-sulfonamide, 2,6-dimethyl-4-methoxybenzenesulfonamide, pentamethyl-benzenesulfonamide, 2,3,5,6-tetramethyl-4-methoxybenzenesulfonamide, 4-methoxybenzenesulfonamide, 2,4,6-trimethylbenzenesulfonamide, 2,6-dimethoxy-4-methylbenzenesulfonamide, 3-methoxy-4-*t*-butylbenzene-sulfonamide, and 2,2,5,7,8-pentamethylchroman-6-sulfonamide. The mention of these groups should be not interpreted as a limitation of the scope of the invention, since they have been mentioned as a mere illustration of protecting groups for amino, but further groups having said function may be known by the skilled person in the art, and they are to be understood to be also encompassed by the present invention.

The term "salt" is to be understood as meaning any form of the compound according to the invention in which this assumes an ionic form or is charged and is coupled with a counter-ion (a cation or anion) or is in solution. By this are also to be understood complexes of the compound with other molecules and ions, in particular complexes which are complexed via ionic interactions. The definition includes in particular physiologically acceptable salts; this term must be understood as equivalent to "pharmacologically acceptable salts" or "pharmaceutically acceptable salts".

The term "physiologically acceptable salt" or "pharmaceutically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄.

For those persons skilled in the art, it will be evident that the scope of the present invention also includes salts which are not pharmaceutically acceptable as possible means for obtaining pharmaceutically acceptable salts.

The term "solvate" according to this invention is to be understood as meaning any form of the compound according to the invention in which this compound has attached to it via non-covalent binding another molecule (most likely a polar solvent) especially including hydrates and alcoholates, e.g. methanolate. Methods of solvation are generally known within the art. The compounds of the invention may present different polymorphic forms, and it is intended that the invention encompasses all such forms.

Any compound that is a prodrug of a compound of formula (I) is also within the scope of the invention. The term "prodrug" is used in its broadest sense and encompasses those derivatives that are converted *in vivo* to the compounds of the invention. Examples of prodrugs include, but are not limited to derivatives of the compounds of formula I that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley), "Design and Applications of Prodrugs" (H. Bundgaard ed., 1985, Harwood Academic Publishers) and Krogsgaard-Larsen et al. "Textbook of Drug design and Discovery" Taylor & Francis (April 2002).

Any compound referred to herein is intended to represent such specific compound as well as certain variations or forms. The compounds of the present invention represented by the above described formula (I) include isomers such as stereoisomers. The term "stereoisomer" as used herein includes any enantiomer, diastereomer or geometric isomer (E/Z) of such compound. In particular, compounds referred to herein may have asymmetric centres and therefore exist in different enantiomeric or diastereomeric forms. Thus any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same or different than the stereoisomerism of the other double bonds of the molecule. All the stereoisomers including enantiomers, diastereoisomers and geometric isomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are enamine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

More particularly, preferred compounds of formula (I) are those having the absolute configuration depicted in formula (I') below 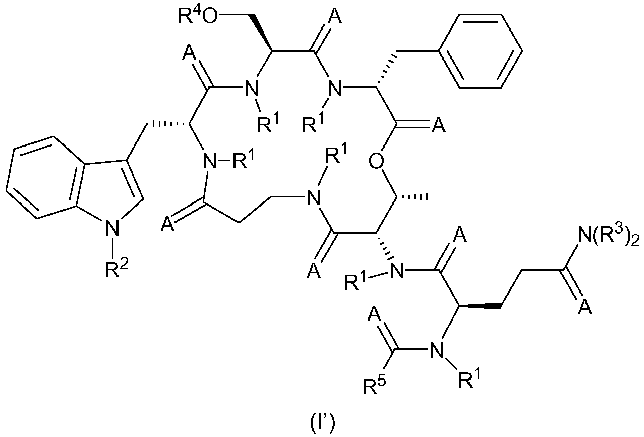 wherein
R¹, R², R³, R⁴, R⁵ and A are as defined above in general formula (I).

According to a particular embodiment, in the compounds of general formula (I) and (I') preferably each R¹, R² and R³ are independently H or an amino protecting group selected from:
- N-alkyl amines and N-aryl amines of formula -R';
- carbamates of formula -C(=O)OR';
- amides of formula -C(=O)R'; and
- N-Si derivatives of formula -Si(R')₃;
wherein R' can be independently selected from substituted or unsubstituted alkyl and substituted or unsubstituted aryl.

More particularly, preferred amino protecting groups are carbamates such as t-butyl carbamate (BOC), benzyl carbamate (CBZ), and 9-flurorenylmethyl carbamate (FMOC).

In a preferred embodiment, all the R¹ are H. In another preferred embodiment, R² is H. In another preferred embodiment, R³ is H. More preferably, all the R¹, R² and R³ are H.

According to a particular embodiment, in the compounds of general formula (I) and (I') preferably R⁴ is H or an hydroxy protecting group selected from:
- ethers of formula -R';
- silyl ethers of formula -Si(R')₃;
- esters of formula -C(=O)R'; and
- carbonates of formula -C(=O)OR';
wherein R' can be independently selected from substituted or unsubstituted alkyl and substituted or unsubstituted aryl.

More particularly, preferred hydroxy protecting groups include ethers such as methyl ether, *tert*-butyl ether, benzyl ether, p-methoxybenzyl ether, 3,4-dimethoxybenzyl ether, trityl ether, allyl ether, methoxymethyl ether, 2-methoxyethoxymethyl ether, benzyloxymethyl ether, p-methoxybenzyloxymethyl ether, 2-(trimethylsilyl)ethoxymethyl, 1-methoxyethyl ether, 1-ethoxyethyl ether, 1-n-propoxyethyl ether, 1-isopropoxyethyl ether, 1-*n*-butoxyethyl ether, 1-isobutoxyethyl ether, 1-sec-butoxyethyl ether, 1-*tert*-butoxyethyl ether, 1-ethoxy-n-propyl ether, methoxypropyl ether, ethoxypropyl ether, 1-methoxy-1-methylethyl ether, 1-ethoxy-1-methylethyl ether; tetrahydropyranyl and related ethers; silyl ethers such as trimethylsilyl ether, triethylsilyl ether, *tert*-butyldimethylsilyl ether *tert*-butyldiphenylsilyl ether, tri-isopropylsilyl ether, diethylisopropylsilyl ether, thexyldimethylsilyl ether, triphenylsilyl ether, di-*tert*-butylmethylsilyl ether, dimethylphenyl ether; esters such as acetate, benzoate, pivaloate, methoxyacetate, chloroacetate, levulinate; carbonates such as benzyl carbonate, p-nitrobenzyl carbonate, *tert*-butyl carbonate, 2,2,2-trichloroethyl carbonate, 2-(trimethylsilyl)ethyl carbonate, allyl carbonate.

In a preferred embodiment, R⁴ is H.

According to a particular embodiment, in the compounds of general formula (I) and (I') preferably R⁵ is C₁-C₁₈ alkyl, more preferably R⁵ is C₆-C₁₈ alkyl and still more preferably R³ is C₁₁ alkyl, particularly linear (unbranched) C₁₁ alkyl.

According to a particular embodiment, in the compounds of general formula (I) and (I') preferably A represents oxygen.

A particularly preferred compound of the invention is the following: 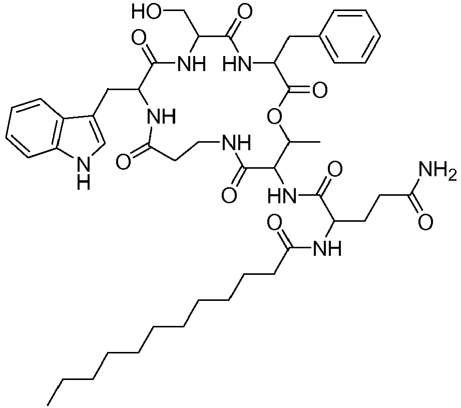 or a pharmaceutically acceptable salt, isomer, prodrug or solvate thereof.

A particularly even more preferred compound of the invention is the following, referred herein to as MDN-0018: 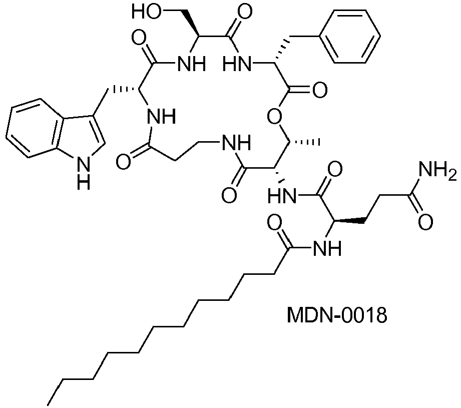 or a pharmaceutically acceptable salt, prodrug or solvate thereof.

In additional preferred embodiments, the preferences described above for the different groups and substituents in the formulae above are combined. The present invention is also directed to such combinations.

MDN-0018 was isolated from the fungus identified as *Colispora cavincola.* This microorganism was isolated from plant litter collected from steppe vegetation near El Calafate, Santa Cruz Province, Argentina. The fungus is deposited in the culture collection of Fundación MEDINA under the accession number CF-226670 and has been also deposited under the Budapest Treaty in culture collection of the Centraalbureau voor Schimmelcultures, Uppsalalaan 83584 CT Utrecht, the Netherlands and has been assigned accession number CBS 133614.

One aspect of the present invention is said strain of *Colispora cavincola* deposited at the CBS with accession number CBS 133614 as well as a culture thereof including a biologically pure culture.

The producer microorganism (CF-226670; CBS 133614) was isolated using a dilution-to-extinction method **(Collado et *al.,* 2007).** On oatmeal agar, the fungus forms velvety to lanose, radially sulcate colonies that attained 35 to 40 mm in 21 days 22 °C and ranged from pale to dark gray, and dark grayish brown in reverse. On 2% malt agar, the colonies attains 25 to 35 mm in diameter in 21 day at 22 °C and ranged from pale gray to dark grayish brown, with dark brown to brownish black submerge mycelium, and a soluble yellowish brown pigment in the agar. Only rarely, on some very nutrient-poor media, e.g. Synthetic Nutrient Agar, was a conidial state evident. The conidia and conidiogenesis were very similar to that previous described for C. *cavincola* (**Gönczöl and Révay, 1996).** The conidia arose singly from terminal cells of unbranched or sparingly branched hyphae. Conidogenesis occurred at the terminal cells by holoblastic secession from the condiogenous locus. A percurrent scar was evident at the conidiogenous locus. The conidia were cylindrical to fusoid, or narrowly clavate, with 0 to 7 transverse septa, with the basal cell being slightly inflated and bearing a secession scar, with dimensions of approximately 20 to 75 µm long and 8 to 15 µm wide, and hyaline to gray in color.

Furthermore to estimate the approximate phylogenetic position of strain CF-226670, genomic DNA was extracted from mycelia grown on malt-yeast extract agar. The rDNA region containing the partial sequence of 28S rDNA containing D1 D2 variable domains was amplified with primers NL1 and NL4 (O'Donnell, 1993) and a DNA sequence was generated. About 0.1 µg/mL of the double-stranded amplification products were sequenced using the ABI PRISM Dye Terminator Cycle Sequencing Ready Reaction Kit (Perkin Elmer, Norwalk CT) following the procedures recommended by the manufacturer. Purified PCR products were directly sequenced using the same primer pairs as in the PCR reactions. Partial sequences obtained in sequencing reactions were assembled with Genestudio 2.1.1.5. (Genestudio, Inc., Suwanee, GA, USA). Database matching with the 28S rDNA sequence (www.fungalbardcoding.org), yielded a very high sequence similarity (99.658%) to the type strain of C. *cavincola* CBS 624.95, thus indicating that strain CF-226670 was genetically similar to C. *cavincola,* and possibly conspecific. High similar scores to other authentic fungi strains, e.g. *Lophiostoma glabrotunicatum* (98.404%), indicated that CF-226670 could be classified as Ascomycota, Pleosporales, and possibly in the family Lophiostomataceae.

MDN-0018 was also isolated from the strain of *Colispora cavincola.*CBS 624.95.

The compound MDN-0018 may be obtained by cultivating C. *cavincola* CF-226670, CBS 133614 or C. *cavincola* CBS 624.95 in a suitable nutrient medium, such as those described below, until a significant amount accumulates in the fermentation. The strain is usually cultured in an aqueous nutrient medium containing sources of assimilable carbon and nitrogen. For example, the cultures may be grown under submerged aerobic conditions (e.g., shaking the culture, submerging the culture, etc.) or in solid state fermentations. The aqueous medium is preferably maintained at a pH of about 6-8, at the initiation and termination (harvest) of the fermentation process. The desired pH may be maintained by the use of a buffer, such as morpholinoethanesulfonic acid (MES), morpholinopropane-sulfonic acid (MOPS), and the like, or by choosing nutrient materials that inherently possess buffering properties. Suitable sources of carbon in the nutrient medium include carbohydrates, such as glucose, xylose, galactose, glycerine, starch, sucrose, dextrin and the like. Other suitable carbon sources that may be used include maltose, rhamnose, raffinose, arabinose, mannose, sodium succinate and the like. Suitable sources of nitrogen are yeast extracts, meat extracts, peptone, gluten meal, cottonseed meal, soybean meal and other vegetable meals (partially or totally defatted), casein hydrolysates, soybean hydrolysates, yeast hydrolysates, corn steep liquor, dried yeast, wheat germ, feather meal, peanut powder, distiller's solubles and the like, as well as inorganic and organic nitrogen compounds, such as ammonium salts (including ammonium nitrate, ammonium sulfate, ammonium phosphate, etc.), urea, amino acids, and the like. The carbon and nitrogen sources, which may be advantageously employed in combination, need not be used in their pure forms; because less pure materials, which contain traces of growth factors, vitamins and significant quantities of mineral nutrients, are also suitable for use. When appropriate, mineral salts, such as sodium or calcium carbonate, sodium or potassium phosphate, sodium or potassium chloride, sodium or potassium iodide, magnesium salts, copper salts, cobalt salts, and the like, may be added to the medium. If necessary, especially when a culture medium foams excessively, one or more defoaming agent(s), such as liquid paraffin, fatty oils, plant oils, mineral oils or silicones, may be added. Submerged aerobic cultural conditions are typical methods of culturing cells for the production of cells in massive amounts. For small-scale production, a shaken or surface culture in a flask or bottle may be employed. When growth is carried out in large tanks, it may be preferable to use the vegetative forms of the organism for inoculation in the production tanks in order to avoid growth lag in the process of production. Accordingly, it may be desirable first to produce a vegetative inoculum of the organism by inoculating a relatively small quantity of culture medium with spores or mycelia of the organism produced in a "slant" or Petri dish and culturing said inoculated medium, also called the "seed medium," and then to transfer the cultured vegetative inoculum aseptically to large tanks. The fermentation medium, in which the inoculum is produced, is generally autoclaved to sterilize the medium prior to inoculation. The pH of the medium is generally adjusted to about 6-7 prior to the autoclaving step. Agitation and aeration of the culture mixture may be accomplished in a variety of ways. Agitation may be provided by a propeller or similar mechanical agitation equipment, by revolving or shaking the fermentor, by various pumping equipment, or by the passage of sterile air through the medium. The fermentation is usually conducted at a temperature between about 20 °C and 30 °C, such as between about 22 °C and 25 °C, for a period of about 7 to 21 days, and parameters may be varied according to fermentation conditions and scales. Preferred culturing/production media for carrying out the fermentation include the media as set forth in the examples

Separation and purification of compounds of the present invention from the crude active extract can be performed using the proper combination of conventional chromatographic techniques.

Additionally, compounds of the invention can be obtained by modifying those already obtained from the natural source or by further modifying those already modified by using a variety of chemical reactions, for instance by means of derivatization, protection/deprotection and isomerisation reactions. Thus, hydroxyl groups can be acylated by standard coupling or acylation procedures, for instance by using acetic acid, acetyl chloride or acetic anhydride in pyridine or the like. Formate groups can be obtained by heating hydroxyl precursors in formic acid. Carbamates can be obtained by heating hydroxyl precursors with isocyanates. Hydroxyl groups can be converted into halogen groups through intermediate sulfonates for iodide, bromide or chloride, or directly using a sulfur trifluoride for fluorides; or they can be reduced to hydrogen by reduction of intermediate sulfonates. Hydroxyl groups can also be converted into alkoxy groups by alkylation using an alkyl bromide, iodide or sulfonate, or into amino lower alkoxy groups by using, for instance, a protected 2-bromoethylamine. Amido groups can be alkylated or acylated by standard alkylation or acylation procedures, for instance by using, respectively, KH and methyl iodide or acetyl chloride in pyridine or the like. Ester groups can be hydrolized to carboxylic acids or reduced to aldehyde or to alcohol. Carboxylic acids can be coupled with amines to provide amides by standard coupling or acylation procedures. When necessary, appropriate protecting groups can be used on the substituents to ensure that reactive groups are not affected. The procedures and reagents needed to prepare these derivatives are known to the skilled person and can be found in general textbooks such as March's Advanced Organic Chemistry 6th Edition 2007, Wiley Interscience. As the skilled person will appreciate, certain compounds of formula (I) may be useful as intermediate products in the preparation of other compounds of formula (I).

An important feature of the above described compounds is their bioactivity and in particular their capacity to potentiate the effect of antifungal drugs. Thus, a further aspect of the present invention relates to a medicament or composition in different pharmaceutical forms comprising at least a compound of formula (I), optionally at least one antifungal drug and at least one pharmaceutically acceptable excipient for use in the treatment and/or prevention of a fungal disease.

A combination of at least a compound of formula (I) and at least one antifungal drug may be formulated for its simultaneous, separate or sequential administration. This has the implication that the combination of the two compounds may be administered:
- as a combination that is being part of the same medicament formulation, the two compounds being then administered always simultaneously.
- as a combination of two units, each with one of the substances giving rise to the possibility of simultaneous, sequential or separate administration.

In a particular embodiment, the compound of formula (I) is independently administered from the antifungal drug (i.e in two units) but at the same time.

In another particular embodiment, the compound of formula (I) is administered first, and then the antifungal drug is separately or sequentially administered.

In yet another particular embodiment, the antifungal drug is administered first, and then the compound of formula (I) is administered, separately or sequentially, as defined.

Examples of pharmaceutical compositions include any solid (tablets, pills, capsules, granules, etc.) semi-solid (creams, ointments, etc.) or liquid (solutions, suspensions or emulsions) composition for oral, topical or parenteral (such as intravenous) administration.

The respective medicament may - depending on its route of administration - also contain one or more excipients known to those skilled in the art. The medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions *(U.S. National Library of Medicine. National Institutes of Health).* Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

As used herein, the terms "treat", "treating" and "treatment" include in general the eradication, removal, reversion, alleviation, modification, or control of a fungal disease after its onset.

As used herein, the terms "prevention", "preventing", "preventive", "prevent" and "prophylaxis" refer to the capacity of a given substance, composition or medicament to avoid, minimize or difficult the onset or development of a fungal disease before its onset.

In particular, examples of fungal diseases which can be treated in the context of the invention are invasive aspergillosis, candidiasis, cryptococcosis, fusariosis, trichosporonosis among other invasive fungal infections as well as fungal infections of the skin, hair, and nails.

Examples of antifungal drugs in the context of the invention include, but are not limited to: echinocandins (caspofungin, micafungin, anidulafungin, enfumafungin), imidazoles (bifonazole, butoconazole, etc.), triazoles (itraconazole, voriconazole, fluconazole, posaconazole, etc.), thiazoles (abafungin), polyenes (amphotericin B, filipin, nystatin, etc.), allylamines (terbinafine, etc.).

Another aspect of the invention is a method of treatment of a patient, notably a human, suffering a fungal disease, or likely to suffer a fungal disease, which comprises administering to the patient in need of such a treatment or prophylaxis an effective amount of a compound of formula (I). Preferably, the method of treatment further comprises administering to the patient an effective amount of an antifungal drug.

By an "effective" amount or a "therapeutically effective amount" of a drug or pharmacologically active agent is meant a nontoxic but sufficient amount of the drug or agent to provide the desired effect. In the combination therapy of the present invention, an "effective amount" of one component of the combination (i.e. compound of formula (I) or antifungal drug) is the amount of that compound that is effective to provide the desired effect when used in combination with the other component of the combination (i.e. antifungal drug or compound of formula (I)). The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

The following examples are merely illustrative of certain embodiments of the invention and cannot be considered as restricting it in any way.

### EXAMPLES

### EXAMPLE 1: FERMENTATION IN ERLENMEYER FLASKS

The potentiating effect of antifungal agents by strain CF-226670 was initially detected in two of a 12-medium nutritional array in 1-mL microfermentations in deepwell 96-well plates **(Bills *et al.,* 2008).** The most potent activity from extracts of a medium designated as Supermalt (malt extract 50 g, yeast extract 10 g, FeSO₄.7H₂O 20 mg, ZnSO₄.7H₂0, 1 L distilled H₂O) was selected for further study. The initial sample for characterization of the compounds potentiating the effect of antifungal agents was prepared by cutting three to four mycelial discs from each 60-mm plate and crushing them in the bottom of tubes (25 × 150 mm) containing 10 mL of Sabouraud maltose broth supplemented with yeast extract and dilute agar (SMYA) (Difco neopeptone 10 g, maltose 40 g, Difco yeast extract 10 g, agar 4 g, distilled H₂O 1000 mL) and two cover glasses (22 × 22 mm). Tubes were agitated on an orbital shaker (200 rpm, 5 cm throw), to produce homogenous hyphal suspensions. After growing the inoculum stage for 4 days, a 0.5 mL aliquot was used to inoculate 15 mL of Supermalt medium in 10 × 50-mL sterile polystyrene tissue culture tubes (Techno Plastic Products AG, Trasadingen, Switzerland). The tubes were incubated statically 21 days at 22 °C. Mycelium and broth from these tubes were extracted with acetone, and after removing the acetone by evaporation, the aqueous residue was used to detect the molecules which potentiate the effect of antifungal agents. To further characterize the molecule responsible for the activity, a 1-L fermentation was prepared. Ten mycelial discs were used to inoculate 50 mL of SMYA. After 4 days, 1-mL aliquots of this culture were used to inoculate Supermalt medium (10 × 100 mL medium in 500-mL Erlenmeyer flasks). The flasks were incubated statically at 22 °C, 70% relative humidity for 21 days. Additional screening of medium formulations indicated that significant amounts of compound MDN-0018 can be produced in MV8 medium (maltose 75 g, V8 juice 200 mL, soy flour 1 g, L-proline 3 g, MES 16.2 g, 800 mL H₂O) in agitated fermentations. Larger amounts of MDN-0018 were prepared by using the same Supermalt medium incubated in an aerated agitated fermentation (220 rpm, 5 cm throw) for 10 to 14 days at 22 °C, 70% relative humidity.

### EXAMPLE 2: ISOLATION AND STRUCTURAL IDENTIFICATION OF MDN-0018.

A 1L culture of CF-226670 *(Colispora cavincola)* grown for 22 days in Supermalt medium was extracted by addition of acetone (1 L), agitation at 220 rpm for 1 h, centrifugation at 8500 rpm, filtration, and evaporation of the organic solvent under a nitrogen stream. The aqueous residue was loaded onto an SP207ss column (65 g, 32 × 100 mm) that was eluted with a gradient of acetone in water (10 to 100% acetone in 12.5 min +100% acetone for 15 min, 8 mL/min, 20 mL/fraction). Fractions 6-8 from this chromatography were pooled and subjected to preparative HPLC (Zorbax SB-C8 PrepHT, 21.2 × 250 mm, 7 µm, gradient H₂O + 0.1 % TFA-CH₃CN + 0.1 % TFA from 5% to 100% organic in 40 min, 20 mL/min, UV detection at 210 and 280 nm). Fractions containing the compound of interest from this chromatography were further purified by semipreparative reversed-phase HPLC (Zorbax RX-C8, 9.4 x 250 mm, gradient H₂O + 0.1 % TFA-CH₃CN + 0.1 % TFA from 5 to 60 % CH₃CN-TFA in 22 min, held at 60 % CH₃CN-TFA for 11 min and from 60 to 100 % CH₃CN-TFA in 2 min, UV detection at 210 and 280 nm) to yield 22 mg of MDN-0018 as a white amorphous solid eluting at 28.8 min.
**MDN-0018:** white amorphous solid; [α]²⁰_{D} (c=0.1, MeOH): +8.47; IR (ATR) v cm⁻¹: 3281, 2925, 2853, 1736, 1543, 1455, 1441, 1234, 1025. HRMS m/z 903.5006 [M+H]⁺ (calcd for C₄₇H₆₇N₈O₁₀, 903.4980); ¹H and ¹³C NMR data see Table 1; UV (DAD) (λₘₐₓ): 219, 279, 291 (sh).

**Table 1. ¹H (500 MHz) and ¹³C (125 MHz) NMR data of MDN-0018 (DMSO-d₆)**

| **amino acid** | **position** | **δ_{c}** | **δ_{H} (mult, *J* in Hz)** | **COSY** | **HMBC (H to C)** |
|---|---|---|---|---|---|
| **D-Phe** | CO | 169.7, C | | | |
| | α | 54.9, CH | 3.74 (m) | β (a, b), NH | CO, Ser-CO, C_{β} |
| | β | 34.8, CH₂ | a: 3.18 (dd, 13.7, 3.9) | α, geminal, 8 (weak) | CO, C_{α}, C_{γ}, C_{δ} |
| | | | b: 3.11 (dd, 13.7, 10.5) | α, geminal, 8 (weak) | CO, C_{α}, C_{γ}, C_{δ} |
| | γ | 138.3, C | | | |
| | δ | 129.3, 2 x CH | 7.18-7.21 (m) | ε, ζ | C_{β}, C_{ζ} |
| | ε | 128.1, 2 x CH | 7.25 (br t, 2H, 7.3) | δ,ζ | Cγ |
| | ζ | 126.1, CH | 7.18-7.21 (m) | δ, ε | C_{δ} |
| | NH | | 7.71 (d, 7.8) | α | Ser-CO, C_{α} |
| | | | | | |
| **L-Ser** | CO | 170.1, C | | | |
| | α | 57.0, CH | 4.04 (m) | β (a), NH | CO, Trp-CO, Cp |
| | β | 60.5, CH₂ | a: 3.62 (dd, 10.8, 6.3) | α, geminal | CO, C_{α} |
| | | | b: 3.52 (dd, 10.8, 2.9) | α, geminal | CO, C_{α} (weak) |
| | NH | | 9.05 (d, 6.4) | α | Trp-CO, C_{α}, Cp |
| | OH | | 4.84 (br s) | | |
| | | | | | |
| **D-Trp** | CO | 173.8, C | | | |
| | α (2) | 54.0, CH | 4.56 (m) | β (a, b), NH | C_{β}, C-4, CO, β-Ala-CO (weak) |
| | β (3) | 27.0, CH₂ | a 3.03 (m) | α, geminal | C_{α}, CO, C-4, C-5, C-11 |
| | | | b 2.88 (dd, 14.5, 10.0) | α, geminal | C_{α}, CO, C-4, C-5, C-11 |
| | 4 | 109.1, C | | | |
| | 5 | 127.0, C | | | |
| | 6 | 118.3, CH | 7.59 (d, 7.8) | H-7, H-8 (weak) | C-4, C-5, C-8, C-9 (weak), C-10 |
| | 7 | 118.2, CH | 6.96 (br t, 7.3) | H-6, H-8 | C-5, C-9 |
| | 8 | 120.9, CH | 7.06 (br t, 7.3) | H-7, H-9 | C-6, C-10 |
| | 9 | 111.3, CH | 7.32 (d, 8.3) | H-8 | C-5, C-7 |
| | 10 | 136.1, C | | | |
| | 11 | 123.5 | 7.08 (br s) | β (a) | C-4, C-5, C-10 |
| | NH (indol) | | 10.84 (s) | | C-4, C-5 |
| | NH | | 7.77 (d, 6.4) | α | β-Ala-CO, C_{α}, Cp |
| | | | | | |
| **β-Ala** | CO | 171.7, C | | | |
| | α | 33.9, CH₂ | a 2.47 (m) | β (a, b), geminal | CO, C*_{β}* |
| | | | b 2.21 (m) | β (b), geminal | CO |
| | β | 36.6, CH₂ | a 3.31 (m) (water-masked) | α (a), geminal, NH | CO, C_{α}, Thr-CO |
| | | | b 2.99 (m) | α (a) (weak), α (b), geminal, NH | CO |
| | NH | | 7.17 (m) | β (a, b) | Thr-CO, C*_{β}* (very weak) |
| **L-Thr** | CO | 168.5, C | | | |
| | α | 55.6, CH | 4.47 (m) | β, NH | CO, C*_{β}* (weak), Gln-CO |
| | | | | | |
| | β | 70.2, CH | 5.36 (br dd, 6.4, 2.9) | α (weak), γ | Cγ, CO (weak), Phe-CO |
| | γ | 15.8, CH₃ | 1.08 (d, 6.4) | β | C_{β}, C_{α} |
| | NH | | 8.50 (m) | α | C_{α}, Gln-CO |
| **D-Gln** | CO | 172.7, C | | | |
| | α | 53.7, CH | 4.49 (m) | β, NH | C_{β}, Cγ |
| | β | 26.8, CH₂ | 1.90 (m, 2H) | α, y | C_{α}, Cγ, CO |
| | γ | 31.5, CH₂ | 2.14 (m, 2H) | β | C_{α}, C_{β}, CO-NH₂ |
| | NH | | 8.48 (m) | α | C_{α}, C_{β}, CO-DDA) |
| | CO-NH₂ | 173.3, C | | | |
| | CO-NH₂ | | a 7.34 (br s) | CO-NH₂ (b) | CO-NH₂ |
| | | | b 6.80 (br s) | CO-NH₂ (a) | Cγ, CO-NH₂ |
| | | | | | |
| DDA | CO | 173.9, C | | | |
| | 2 | 34.7, CH₂ | 2.19 (t, 7.3), 2H | 3 | C-3, C-4 |
| | 3 | 25.0, CH₂ | 1.47 (m), 2H | 2, 4 | C-2, C-4 |
| | 4 | 28.5, CH₂ | 1.15-1.28 (m) | 3 | C-3 |
| | 5 | 29.0, CH₂ * | 1.15-1.28 (m) | | |
| | 6 | 28.9, CH₂ * | 1.15-1.28 (m) | | |
| | 7 | 28.8, CH₂ * | 1.15-1.28 (m) | | |
| | 8 | 28.7, CH₂ * | 1.15-1.28 (m) | | |
| | 9 | 28.6, CH₂ * | 1.15-1.28 (m) | | |
| | 10 | 31.2, CH₂ | 1.15-1.28 (m) | | |
| | 11 | 22.0, CH₂ | 1.24 (m, 2H) | 10, 12 | C-10, C-12 |
| | 12 | 13.9, CH₃ | 0.84 (t, 7.1) | 11 | C-11, C-10 |

| | | | | | |
|---|---|---|---|---|---|
| *Assignments are interchangeable DDA: dodecanoic acid | | | | | |

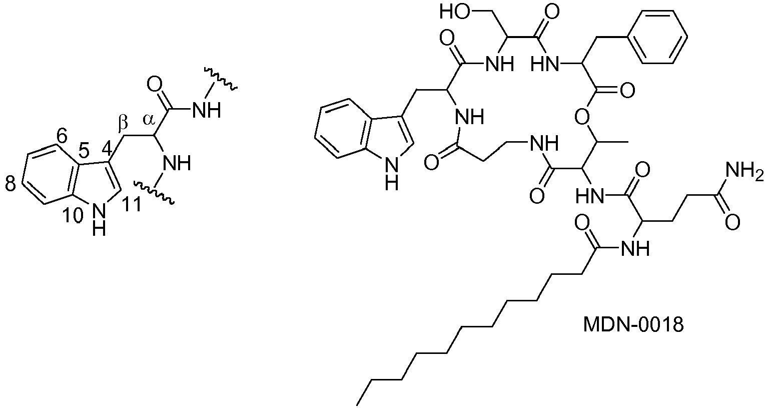

### EXAMPLE 3: DETERMINATION OF THE ABSOLUTE CONFIGURATION OF THE AMINO ACID RESIDUES IN MDN-0018

Marfey's analysis **(Marfey, 1984)** was used to determine the absolute configuration of the amino acid residues in MDN-0018.

220 µg of MDN-0018 were dissolved in 0.44 mL of 6N HCl and heated at 110 °C for 16 h. The crude hydrolizate was evaporated to dryness under a N₂ stream and the residue was dissolved in 100 µL of water.

A 1% (w/v) solution (100 µL) of L-FDVA (Marfey's reagent, N-(2,4-dinitro-5-fluorophenyl)-L-valinamide) in acetone was added to an aliquot (50 µL) of a 50 mM solution of each amino acid (D, L, or DL mixture) and to the aqueous solution of the peptide hydrolizate. After addition of 20 uL of 1 M NaHCO₃ solution, each mixture was incubated at 40 °C for 60 min. The reactions were quenched by addition of 10 µL of 1 N HCl and the crude mixtures were diluted with 700 µL of acetonitrile and analyzed by ESI LC/MS.

HPLC analyses were performed on an Agilent 1100 single Quadrupole LC/MS. Separations were carried out on a Waters XBridge C18 (4.6 x 150 mm, 5 µm) maintained at 40°C. A mixture of two solvents, A (10% acetronitrile, 90% water) and B (90% acetronitrile, 10% water), both containing 1.3 mM trifluoroacetic acid and 1.3 mM ammonium formate, was used as the mobile phase under a linear gradient elution mode (10 to 30% B in 35 min, 30 to 100 % B in 1 min, isocratic 100 % B for 4 min) at a flow rate of 1 mL/min.

Retention times (min) for the derivatized (L-FDVA) amino acid standards under the reported conditions were as follows: L-Phe: 34.66; D-Phe: 37.87; L-Ser: 12.63; D-Ser: 14.94; L-Trp: 35.33; D-Trp: 37.72; L-Thr: 13.02; D-Thr: 22.88, L-allo-Thr: 13.83; D-allo-Thr: 16.14; L-Glu: 15.42; D-Glu: 19.59.

Retention times (min) for the observed peaks in the HPLC trace of the L-FDVA derivatized hydrolysis product of MDN-0018 were as follows: D-Phe: 37.85; L-Ser: 12.63; D-Trp: 37.72; L-Thr: 13.01; D-Glu: 19.62.

Compound MDN-0018 has therefore the following absolute configuration:

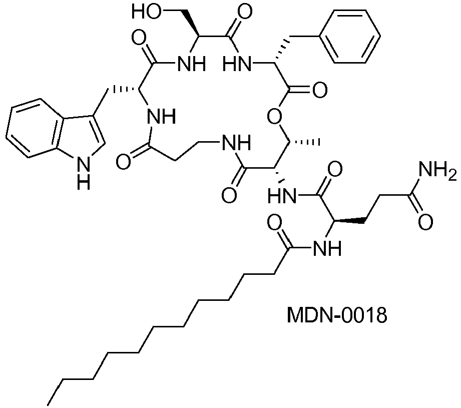

### EXAMPLE 4: ASSESMENT OF THE POTENTIATION OF ANTIFUNGAL ACTIVITY BY THE COMPOUND MDN-0018

For the evaluation of the potentiation of the antifungal activity by the compound MDN-0018 the checkerboard methodology was applied. This method is used to compare the *in vitro* efficacies of different antimicrobial combinations (Fig. 1 shows the checkboard of MDN-0018 and caspofungin; similar results were obtained with other combinations).

The checkerboard microtiter plate assay tests the activities of several drugs in combination against different strains by determining the best drug combination among the tested samples. The effect of combination in each assay is displayed as dose-response curves of know antifungal drugs in the microtiter plate columns and dose-response curves of the MDN-0018 compound in the microtiter plate rows.

The checkerboard assay evaluated the combinations effects with different fungal microorganisms, including *Aspergillus fumigatus, Candida albicans, Candida glabrata, Cryptococcus neoformans, Saccharomyces cerevisiae* among others.

The checkerboard assay was performed with different commercial antifungal drugs such as echinocandins (caspofungin, micafungin, anidulafungin), azoles (itraconazol, voriconazol, fluconazole), amphotericin B, along with others. The cellular viability was scored via resazurin and the methodology used was as described by **Monteiro et *al.,* 2012.**

The checkerboard results showed that compound MDN-0018 potentiates the effect of the different known antifungals in 2.5 to 8.5 times depending on the microorganism and the known antifungal tested. An example of this potentiation (MDN-0018 and caspofungin against *A. fumigatus)* is graphically represented in Figure 2.

**Table 2. Study of the optimum concentration of the compounds MDN-0018 and caspofungin for maximum enhancement of caspofungin.**

| ***A. fumigatus* ATCC 46645** | | |
|---|---|---|
| | **Compound [µM]** | **Combination [IC₅₀µM]** |
| | 8.8600 | 0.0064 |
| | 4.4300 | 0.0084 |
| **Optimal** | **2.2150** | **0.0068** |
| | 1.1075 | 0.0241 |
| | 0.5538 | 0.0256 |
| | 0.2769 | 0.0323 |
| | 0.1384 | 0.0302 |
| | 0.0692 | 0.0337 |
| | 0.0346 | 0.0359 |
| | 0.0173 | 0.0343 |

### Caspofungin Potentiation 5.3 times

### EXAMPLE 5: ASSESSMENT OF CELL VIABILITY USING PROBE FOR MEMBRANE INTEGRITY

Cell viability was determined by changes in membrane permeability, live cells with intact membranes are distinguished by their ability to exclude dyes that easily penetrate dead or damaged cells. A fluorescent staining, DNA-binding probe propidium iodide (PI) was used to indicate non viable cells by dye exclusion (indicative of an intact membrane).

*C. albicans* was tested along with different known antifungal compounds (caspofungin, amphotericin B and azoles) to demonstrate the lack of activity of these antifungals at the sublethal doses used. Compound MDN-0018 by itself did not have antifungal effect when tested at concentrations below 8 µg/mL. However, MDN-0018 in combination with sublethal doses of these antifungals was able to cause cell damage and completely inhibit the growth in some occasions. Figure 3 shows an example of the results obtained using caspofungin.

Flow cytometry experiments were performed with MDN-0018 using PI stain as probe. The effects shown in the Figure 3 demonstrated that compound MDN-0018 does not affect the membrane permeability given that the PI does not penetrate in the cell and the cellular population remains the same size and complexity as the population not treated with the stain.

### REFERENCES:

- Endo EH, Cortez DA, Ueda-Nakamura T, Nakamura CV, Dias Filho BP. 2010. Potent antifungal activity of extracts and pure compound isolated from pomegranate peels and synergism with fluconazole against Candida albicans. Res Microbiol; 161 (7):534-40.
- Faria NC, Kim JH, Gonçalves LA, Martins Mde L, Chan KL, Campbell BC. 2011. Enhanced activity of antifungal drugs using natural phenolics against yeast strains of Candida and Cryptococcus. Lett Appl Microbiol; 52(5):506-13.
- Harris MR, Coote PJ. 2010. Combination of caspofungin or anidulafungin with antimicrobial peptides results in potent synergistic killing of Candida albicans and Candida glabrata in vitro. Int J Antimicrob Agents; 35(4):347-56.
- Arikan S, Lozano-Chiu M, Paetznick V, Rex JH. 2002. In vitro synergy of caspofungin and amphotericin B against Aspergillus and Fusarium spp. Antimicrob Agents Chemother; 46(1):245-7.
- Kiraz N, Dag I, Yamac M, Kiremitci A, Kasifoglu N, Oz Y. 2010. Synergistic activities of three triazoles with caspofungin against Candida glabrata isolates determined by time-kill, Etest, and disk diffusion methods. Antimicrob Agents Chemother. 35(4):347-56.
- Collado J, Platas G, Paulus B, Bills GF. 2007 High-throughput culturing of fungi from plant litter by a dilution-to-extinction technique.FEMS Microbiol Ecol; 60(3):521-33.
- Gönczöl J, Révay Á. 1996. A new species of Colispora (Hyphomycetes) from tree hollows. Mycotaxon 59: 237-244.
- O'Donnell K, 1993. Fusarium and its near relatives. In: Reynolds DR, Taylor JW (eds). The fungal holomorph: mitotic, meiotic and pleomorphic speciation in fungal systematic, CAB international, Wallingford, UK, pp. 225-233.
- Bills G, Platas G, Fillola A, Jiménez MR, Collado J, Vicente F, Martín J, González A, Bur-Zimmermann J, Tormo JR & Peláez F. 2008. Enhancement of antibiotic and secondary metabolite detection from filamentous fungi by growth on nutritional arrays. Journal of Applied Microbiology 104:1644-1658.
- Marfey P. Carlsberg Res. Commun. 1984, 49, 591-596
- Monteiro MC, de la Cruz M, Cantizani J, Moreno C, Tormo JR, Mellado E, De Lucas JR, Asensio F, Valiante V, Brakhage AA, Latgé JP, Genilloud O, Vicente F. 2012. A new approach to drug discovery: high-throughput screening of microbial natural extracts against Aspergillus fumigatus using resazurin. J Biomol Screen; 17(4):542-9.

## Claims

1. A compound of general formula (I) or a pharmaceutically acceptable salt, isomer, prodrug or solvate thereof 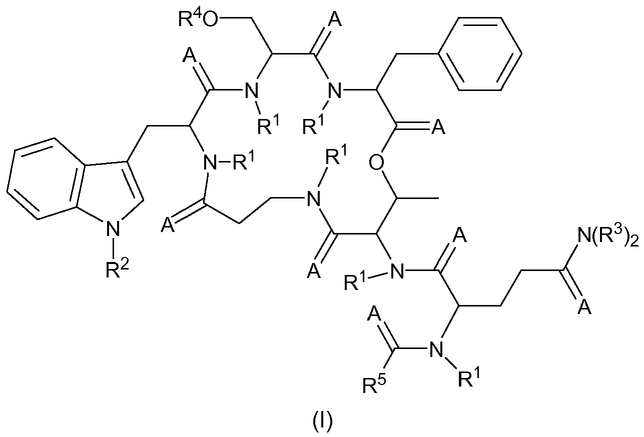 wherein
each R¹, R² and R³ are independently selected from hydrogen and an amino protecting group;
R⁴ is selected from hydrogen and a hydroxyl protecting group;
R⁵ is selected from hydrogen, ORₐ, OCORₐ, NRₐR_{b}, substituted or unsubstituted C₁-C₁₈ alkyl, substituted or unsubstituted C₂-C₁₈ alkenyl, substituted or unsubstituted C₂-C₁₈ alkynyl, and substituted or unsubstituted aryl;
each A is independently selected from oxygen and sulphur, and
each Rₐ and R_{b} is independently selected from hydrogen, substituted or unsubstituted C₁-C₁₂ alkyl, substituted or unsubstituted C₂-C₁₂ alkenyl, substituted or unsubstituted C₂-C₁₂ alkynyl, and substituted or unsubstituted aryl.

2. Compound according to claim 1, wherein each R¹, R² and R³ are independently H or an amino protecting group selected from:
- N-alkyl amines and N-aryl amines of formula -R';
- carbamates of formula -C(=O)OR';
- amides of formula -C(=O)R'; and
- N-Si derivatives of formula -Si(R')₃;
wherein R' can be independently selected from substituted or unsubstituted alkyl and substituted or unsubstituted aryl.

3. Compound according to claim 2, wherein R¹, R² and R³ are H.

4. Compound according to any one of claims 1 to 3, wherein R⁴ is independently H or an hydroxy protecting group selected from:
- ethers of formula -R';
- silyl ethers of formula -Si(R')₃;
- esters of formula -C(=O)R'; and
- carbonates of formula -C(=O)OR';
wherein R' can be independently selected from substituted or unsubstituted alkyl and substituted or unsubstituted aryl.

5. Compound according to claim 4, wherein R⁴ is H.

6. Compound according to any one of claims 1 to 5, wherein R⁵ is C₁-C₁₈ alkyl, preferably wherein R⁵ is C₁₁ alkyl.

7. Compound according to any one of claims 1 to 6, wherein A is oxygen.

8. Compound according to any one of claims 1 to 7, which has the following structure: 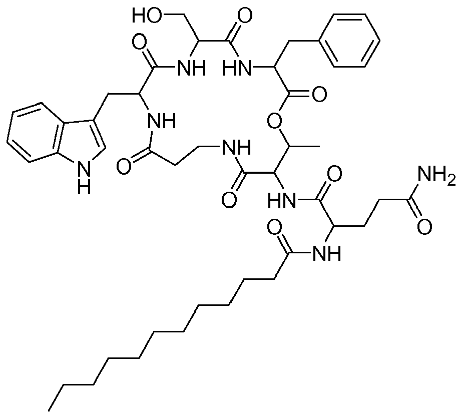 or a pharmaceutically acceptable salt, isomer, prodrug or solvate thereof, preferably which has the following structure: 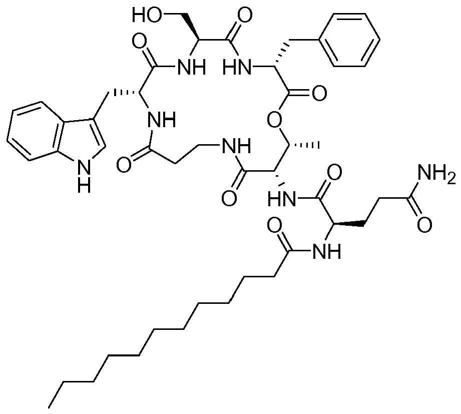 or a pharmaceutically acceptable salt, prodrug or solvate thereof.

9. Pharmaceutical composition comprising at least one compound of formula (I) as defined in any one of claims 1 to 8, or a pharmaceutically acceptable salt, isomer, prodrug or solvate thereof and a pharmaceutically acceptable excipient.

10. Pharmaceutical composition according to claim 9 which further comprises an antifungal drug.

11. Compound according to any one of claims 1 to 8 for use in medicine.

12. Compound according to any one of claims 1 to 8 for use in the treatment and/or prophylaxis of a fungal disease.

13. Compound according to any one of claims 1 to 8 for use in potentiating the effect of antifungal drugs.

14. A strain of *Colispora cavincola* deposited at the CBS with accession number CBS 133614.

15. A process for obtaining a compound of general formula (I) as defined in any one of claims 1 to 8 or a pharmaceutically acceptable salt, isomer, prodrug or solvate thereof, said process comprising the steps of cultivating the strain of C. *cavincola* CBS 133614 or CBS 624.95 in an aqueous nutrient medium with assimilable carbon and nitrogen sources and salts, under controlled submerged aerobic conditions, and then recovering and purifying the compound of general formula (I) from the cultured broth.
